# FASCICULE DE BREVET EUROPEEN

(11) **EP 1 731 194 B1**
(45) Date de publication et mention de la délivrance du brevet: **27.07.2011**
(21) Numéro de dépôt: 06290943.7
(22) Date de dépôt: 09.06.2006
(51) Int. Cl.: A61N 1/362

(54) **Dispositif médical implantable actif tel que stimulateur cardiaque, cardioverteur et/ou défibrillateur de type AAI ou AAI/DDD à détection des tachycardies ventriculaires**
Aktive implantierbare medizinische Vorrichtung, insbesondere Herzschrittmacher, Defibrillator, und/oder Kardiovertierer AAI oder AAI/DDD mit Erfassung von ventrikulären Tachykardien
Active implantable medical device such as pacemaker, defibrillator and/or cardiovertor of type AAI or AAI/DDD, with ventricular tachycardia detection means

(30) Priorité: 09.06.2005 FR 0505851
(43) Date de publication de la demande: 13.12.2006
(73) Titulaire: ELA MEDICAL, 92541 Montrouge (FR)
(72) Inventeur: Amblard, Amel, 92290 Chatenay Malabry (FR); Hallier, Benoît, 1050 Bruxelles (FR)
(74) Mandataire: Dupuis-Latour, Dominique

(56) Documents cités:
- EP-A- 1 470 836
- US-A- 5 814 083
- US-A1- 2003 088 288
- US-A1- 2004 111 121
- US-B1- 6 871 097

## Description

L'invention concerne les "dispositifs médicaux implantables actifs" tels que définis par la directive 90/385/CEE du 20 juin 1990 du Conseil des communautés européennes, et plus précisément les dispositifs stimulateurs cardiaques, dispositifs "multisite" (triple ou quadruple chambre), défibrillateurs et/ou cardioverteurs, permettant de surveiller l'activité cardiaque et de délivrer au coeur des impulsions électriques de stimulation, de resynchronisation, de cardioversion et/ou de défibrillation en cas de trouble du rythme diagnostiqué par l'appareil.

Elle concerne plus particulièrement ceux de ces dispositifs qui comprennent des circuits de stimulation et de détection à la fois sur l'oreillette et sur le ventricule et qui peuvent opérer selon deux modes de fonctionnement, DDD ou AAI (le mode AAI étant en fait un mode DDD modifié par allongement du délai auriculo-ventriculaire). Ces dispositifs peuvent être pourvus d'un mode dénommé "DDD-CAM" ou "AAISafeR" assurant une commutation automatique de mode (CAM) de DDD en AAI et inversement.

Le mode de fonctionnement de base d'un stimulateur DDD/AAI est un mode AAI - ou plus précisément "pseudo-AAI" - avec stimulation auriculaire simple chambre (mode AAI *stricto sensu)* et surveillance de l'activité ventriculaire. Ce mode est en principe maintenu tant que la conduction auriculo-ventriculaire est normale, c'est-à-dire tant que chaque événement auriculaire (détection auriculaire, correspondant à une activité spontanée, ou stimulation auriculaire) est suivi d'une détection ventriculaire associée.

Dans certaines circonstances peuvent cependant apparaître des blocs auriculo-ventriculaires (BAV) entraînant un trouble temporaire de dépolarisation du ventricule. Dans ce cas, dès lors qu'un certain nombre de conditions sont remplies, le stimulateur bascule automatiquement en mode DDD automatique, avec des paramètres optimisés pour cette situation de BAV temporaire. Après disparition du BAV, et donc rétablissement de la conduction auriculo-ventriculaire, le stimulateur retourne automatiquement au mode AAI dès lors qu'un certain nombre d'autres conditions sont remplies.

Comme on le verra par la suite, l'invention n'est toutefois pas limitée à ces dispositifs à commutation automatique de mode, mais peut s'appliquer à des dispositifs opérant en mode AAI seul, dans la mesure où l'invention propose seulement d'aménager le fonctionnement du mode AAI sans qu'il y ait commutation automatique de mode, et/ou pour éviter une commutation automatique vers le mode DDD.

Le EP-A-1 470 836 (ELA Medical) décrit un tel dispositif stimulateur de type AAI ou DDD/AAI.

Le point de départ de l'invention réside dans un certain nombre d'observations effectuées lors d'un suivi clinique de patients appareillés avec de tels dispositifs.

II est apparu en effet que ces dispositifs ne réagissent pas de façon satisfaisante lorsqu'apparaissent certaines formes de tachycardies ventriculaires (TV) de fréquence relativement stable et voisine du double de la fréquence de stimulation auriculaire.

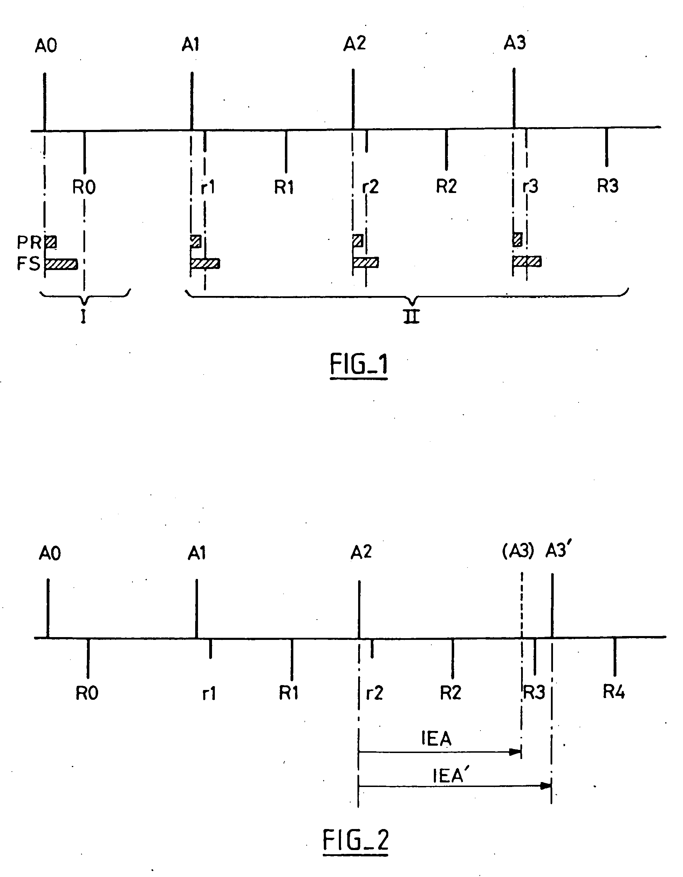

En effet, comme on l'expliquera plus loin dans la description détaillée en référence à la figure 1, la "fenêtres de sécurité" consécutive à chaque stimulation auriculaire masque un évènement ventriculaire sur deux. De la sorte, le dispositif voit un rythme ventriculaire de fréquence moitié de la fréquence réelle. De ce fait, outre la non-détection du trouble ventriculaire lié à cette tachycardie, le fonctionnement du dispositif est perturbé, ce qui peut entraîner un faux diagnostic provoquant une commutation inopportune du dispositif en mode DDD. Bien qu'un fonctionnement en mode DDD n'ait généralement pas de conséquence délétère pour le patient, une telle commutation est inutile et empêche l'expression de la conduction auriculo-ventriculaire spontanée, ce qui peut bloquer le diagnostic d'autres troubles du rythme ventriculaire, en.

Les observations cliniques montrent également qu'un tel fonctionnement non conforme dû au masquage d'évènements ventriculaires peut également survenir lorsqu'en présence d'un BAV se manifeste un phénomène connu sous le nom de "cross-talk" ou "écoute croisée" auriculo-ventriculaire (CTAV). Ce phénomène se produit lorsque le stimulateur détecte dans la cavité ventriculaire un signal provenant en fait du stimulus électrique auriculaire distant. Une telle situation doit pouvoir être détectée et caractérisée pour diagnostiquer l'apparition effective d'un BAV.

L'invention a pour but de remédier aux inconvénients précités, en proposant un dispositif permettant de démasquer certains types de TV susceptibles de leurrer les algorithmes du dispositif, et également de caractériser ces TV en les distinguant des situation de CTVA susceptibles de produire des signaux comparables.

Le dispositif de l'invention est un dispositif de type AAI ou AAI/DDD du type général enseigné par le EP-A-1 470 836 précité, correspondant au préambule de la revendication 1. Pour atteindre les buts énoncés plus haut, l'invention est caractérisée par les éléments énoncés dans la partie caractérisante de la revendication 1. 1.

les sous-revendications énoncent des formes de mise en oeuvre subsidiaires avantageuses.

On va maintenant décrire un exemple de mise en oeuvre de l'invention, en référence aux dessins annexés.
La figure 1 est un chronogramme illustrant le masquage de certains évènements ventriculaires dans le cas d'une TV stable de fréquence double de celle de la stimulation auriculaire.
La figure 2 illustre la manière dont la modification de l'intervalle d'échappement auriculaire permet de démasquer les dépolarisations auparavant masquées, et confirmer ainsi la présence d'une TV.

L'invention peut être mise en oeuvre par une programmation appropriée du logiciel de commande d'un dispositif connu, par exemple les dispositifs implantables *Symphony et ELA Rhapsody* commercialisés par ELA Médical, Montrouge, France. Il s'agit de dispositifs à microprocesseur programmable comportant des circuits pour recevoir, mettre en forme et traiter des signaux électriques recueillis par des électrodes implantées et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

Sur la figure 1, les marqueurs A0, A1, A2, A3 ... illustrent la position dans le temps des impulsions de stimulation auriculaires successives, caractéristiques d'un fonctionnement en mode AAI.

En cas de conduction auriculo-ventriculaire normale, c'est-à-dire en l'absence de BAV, le dispositif détecte une dépolarisation ventriculaire consécutive R0 (cas I).

Plus précisément, après délivrance d'une impulsion de stimulation, le dispositif applique concurremment aux circuits de détection ventriculaire :
- une période réfractaire PR pendant laquelle est opéré un "blanking" ou déconnexion des circuits de détection de manière à masquer les perturbations des amplificateurs juste après la stimulation (perturbations dues à l'écoulement des charges à l'interface électrode/myocarde) ; et
- une période FS dite "fenêtre de sécurité", typiquement d'une durée de 94 ms à partir de la stimulation auriculaire.

La période réfractaire PR peut avantageusement comprendre une période réfractaire absolue de 16 ms, suivie d'une période de recherche de bruit de 16 ms redéclenchable par pas de 2 ms et limitée à 32 ms - c'est-à-dire que cette période réfractaire pourra durer de 32 à 48 ms après la stimulation auriculaire. Pour plus de détails sur la manière dont est gérée cette période réfractaire, on pourra se référer au EP-A-0 962 235 (ELA Medical).

Après écoulement de la période réfractaire PR, les circuits de détection ventriculaires sont fonctionnels, c'est-à-dire qu'il est possible de détecter les dépolarisations ventriculaires survenant pendant ce laps de temps. Toutefois, si une dépolarisation est détectée avant la fin de la fenêtre de sécurité FS, elle est ignorée par le dispositif car, compte tenu du délai très court la séparant de la stimulation auriculaire antérieure, il s'agit vraisemblable d'un artéfact ou d'une extrasystole ventriculaire (ESV) isolée, dont la prise en compte pourrait perturber le déroulement normal de l'algorithme d'analyse du rythme.

Pour cette raison, dans les dispositifs de la technique antérieure, une telle dépolarisation est systématiquement ignorée et n'est pas considérée comme la fin (ou le début) d'un cycle ventriculaire, notamment pour ce qui concerne:
- la gestion des intervalles d'échappement et du délai ventriculo-auriculaire,
- les fonctions de détection des arythmies ventriculaires lorsque le dispositif en est équipé, notamment la discrimination entre tachycardie et fibrillation ventriculaire,
- la gestion de la commutation éventuelle du mode AAI vers le mode DDD.

L'invention vise la situation particulière où le fonctionnement du dispositif peut être pris en défaut lorsque le rythme ventriculaire spontané s'accélère jusqu'à caractériser une TV, ci-après désignée "TV stable 2:1", présentant certains paramètres spécifiques, plus précisément une TV :
- de fréquence stable : par "fréquence stable" ou "intervalle stable", on entendra une fréquence ou une durée dont les valeurs successives sont comprises dans une plage définie à x %, ou x ms, ou x cpm autour d'une valeur nominale ; cette analyse de stabilité peut notamment être opérée par l'algorithme d'analyse du rythme ventriculaire décrit dans le EP-A-0 838 235 (ELA Medical) ;
- de fréquence approximativement double de celle de la fréquence de stimulation auriculaire ; et
- dont une dépolarisation sur deux survient dans un délai compris entre la fin de la période réfractaire PR et la fin de la fenêtre de sécurité FS, ce qui peut arriver notamment lorsque la TV a commencé sur une ESV entraînant un intervalle de couplage long.

Cette situation est illustrée en II sur la figure 1, où les événements ventriculaires successifs apparaissent en r1, R1, r2, R2, r3, R3 ... Les événements R1, R2, R3 ... sont détectés par le dispositif et reconnus comme telles ; en revanche, les événements r1, r2, r3 ... sont masqués par la fenêtre de sécurité FS, c'est-à-dire qu'ils sont bien détectés par le dispositif (puisqu'ils surviennent après la fin de la période réfractaire PR), mais ne sont pas reconnus comme tels car survenant à l'intérieur de la fenêtre de sécurité FS.

En d'autres termes, la fenêtre de sécurité des stimulations auriculaires A1, A2, A3 ... masque un évènement ventriculaire sur deux.

Cette situation présente un triple inconvénient :
- tout d'abord, la tachycardie n'est pas détectée comme telle, car le dispositif mesure le rythme ventriculaire sur la base des évènements non masqués, et calcule donc une fréquence ventriculaire qui est la moitié de la fréquence réelle.
- le délai auriculo-ventriculaire (DAV) déterminé par le dispositif, c'est-à-dire l'intervalle de temps [A1 R1], [A2 R2], [A3 R3] ... semble long (par rapport au délai réel [A1 r1], [A2 r2], [A3 r3] ...), ce qui peut leurrer le dispositif en lui faisant croire à un trouble de la conduction correspondant à un BAV de type BAV1, trouble caractérisé par un délai de conduction auriculo-ventriculaire supérieur à un seuil donnée. Dans un tel cas, un dispositif à commutation automatique de mode déclenche rapidement une commutation en mode DDD, par exemple après six cycles consécutifs vérifiant cette condition.
- lorsque le dispositif comporte une fonction de détection des arythmies ventriculaires (comme décrit dans le EP-A-1 400 260 précité), le fait que la TV ne soit pas reconnue entraîne une anomalie de fonctionnement, le dispositif voyant - à tort - un rythme lent, physiologique. Cette situation est susceptible de conduire à un retard de détection ou à une sous-détection des arythmies, avec par voie de conséquence retard ou non-application d'une thérapie appropriée (stimulation anti-tachy-cardique à haute fréquence de type "ATP" ou choc de défibrillation ou de cardioversion).

Le phénomène décrit ci-dessus peut également survenir lorsqu'en présence d'un BAV se manifeste un cross-talk auriculo-ventriculaire (CTAV), le dispositif voyant des dépolarisations ventriculaires là où il n'y a en fait que des détections dans le ventricule de signaux provenant des stimuli auriculaires.

L'invention propose de remédier à ces inconvénients, en analysant la séquence des évènements ventriculaires recueillis pour y détecter une éventuelle TV stable 2:1 afin d'assurer une détection et un traitement rapide de la TV et éviter un basculement inutile vers le mode DDD.

La première étape est une suspicion de TV stable 2:1 en mode AAI.

Cette suspicion correspond aux quatre conditions suivantes :
a) détection d'un motif caractéristique de type A1 - r1 - R1 - A2 - r2 - R2, c'est à dire dans lequel, au cours d'un même cycle auriculaire, une stimulation auriculaire (A1 ; A2) est suivie de deux détections d'évènements ventriculaires (r1, R1 ; r2, R2), l'une en fenêtre de sécurité (r1; r2), l'autre hors fenêtre de sécurité (R1 ; R2) ;
b) optionnellement, lorsque le dispositif comporte des fonctions de détection des arythmies ventriculaires, on vérifie que la durée de l'intervalle de couplage ventriculaire (intervalles [R1 r2], [r2 R2] ...) est plus courte que la valeur programmée de détection des TV, c'est-à-dire qu'il y a eu franchissement du seuil de détection des TV, seuil connu dans la technique sous le nom de "seuil TDI" *(Tachycardia Detection Interval*) ; on pourra se référer au EP-A-1 400 260 précité pour plus de détails sur cette technique de détection ;
c) stabilité des intervalles de couplage ventriculaire [R1 r2], [r2 R2] ..., par exemple stabilité à ± x ms près, de manière à s'assurer que le motif caractéristique perdure ;
d) une modulation de l'IEA (voir ci-dessous) n'est pas intervenue au cours des y derniers cycles, par exemple au cours des 20 derniers cycles ; ce critère permet de limiter le nombre de modulations lorsque l'on est en présence du cas exposé plus haut d'un BAV avec CTAV.

Lorsque les conditions de suspicion ci-dessus sont vérifiées, pour avérer la présence d'une TV stable 2:1 le dispositif module l'intervalle d'échappement auriculaire (IEA) de manière à autoriser la détection de deux dépolarisations ventriculaires consécutives hors période réfractaire au cours d'un même cycle auriculaire (l'intervalle d'échappement auriculaire (IEA) est l'intervalle de temps, compté après une détection ou une stimulation dans l'oreillette, à l'issue duquel une stimulation est délivrée à cette oreillette si aucun évènement spontané n'a été détecté dans l'oreillette). Si tel est le cas, le dispositif confirme la présence de la TV stable 2:1.

Cette modulation de l'IEA est illustrée sur la figure 2 : au cycle auriculaire commençant en A2, avec l'IEA courant l'impulsion auriculaire est appliquée à l'instant A3, de sorte que l'évènement ventriculaire r3, postérieur à A3, est masqué par la période réfractaire déclenchée sur la stimulation A3, comme cela avait été décrit plus haut en référence à la figure 1. Si l'IEA est allongé pour donner IEA', ceci a pour effet de retarder en A3' la stimulation auriculaire, de sorte que l'évènement ventriculaire suivant l'évènement R2 va apparaître avant la fin de l'IEA, donc avant A3' et hors fenêtre de sécurité de A2, et sera donc pris en compte comme tel par le dispositif. En d'autres termes, l'allongement de l'IEA permet de démasquer l'évènement ventriculaire R3, autrefois masqué en r3 par la fenêtre de sécurité.

L'allongement de l'IEA est une durée fixe, par exemple de 125 ms, valeur avantageusement programmable.

L'évènement R3 démasqué par l'allongement de l'IEA étant considéré comme une ESV, l'IEA est recyclé sur cet évènement. Par ailleurs, dès que la TV a été démasquée, l'IEA est restauré à sa valeur antérieure, pour que le dispositif puisse retourner à son fonctionnement normal.

Le dispositif peut ensuite prendre en compte la TV stable 2:1 ainsi démasquée, par exemple pour appliquer une thérapie appropriée telle qu'une stimulation antitachycardique à haute fréquence.

Par ailleurs, dans le cas d'un dispositif à commutation automatique de mode, le basculement du mode AAI en mode DDD est inhibé par la détection de la TV, le dispositif continuant à analyser le rythme pour y détecter d'éventuels BAV vrais.

## Revendications

1. Un dispositif médical implantable actif de type prothèse cardiaque de stimulation, resynchronisation, cardioversion et/ou défibrillation, ce dispositif étant apte à opérer dans au moins un mode de fonctionnement AAI avec détection ventriculaire et comportant:
- des moyens de détection d'événements ventriculaires et auriculaires spontanés;
- des moyens de délivrance d'impulsions de stimulation auriculaire;
- des moyens aptes, après délivrance d'une impulsion de stimulation auriculaire, à appliquer concurremment aux moyens de détection ventriculaire une période réfractaire (PR) de durée prédéterminée,
ce dispositif étant **caractérisé en ce qu'**il comporte en outre:
- des moyens aptes, après délivrance d'une impulsion de stimulation auriculaire, à appliquer concurremment aux moyens de détection ventriculaire une fenêtre de sécurité (FS) de durée prédéterminée;
- des moyens de détermination d'un début de cycle ventriculaire spontané en réponse à une détection d'événement ventriculaire hors fenêtre de sécurité (R0, R1, R2, R3);
- des moyens de détection de tachycardies ventriculaires en réponse à la détection d'événements ventriculaires survenant à la fois en fenêtre de sécurité (r1, r2, r3) et hors fenêtre de sécurité (R0, R1, R2, R3).

2. Le dispositif de la revendication 1, dans lequel :
- le dispositif est également apte à opérer dans un mode de fonctionnement DDD et comporte des moyens aptes à commander le basculement conditionnel du mode AAI vers le mode DDD, et inversement, et
- les moyens de détection de tachycardies ventriculaires sont aptes à inhiber le basculement du mode AAI vers le mode DDD en cas de détection d'une tachycardie ventriculaire.

3. Le dispositif de la revendication 1, dans lequel :
- les moyens de détection de tachycardies ventriculaires comprennent des moyens de suspicion préalable de tachycardie ventriculaire stable, conditionnés par :
a) la détection d'une séquence d'événements comprenant, entre deux stimulations auriculaires consécutives (A1, A2 ; A2, A3), un événement ventriculaire survenant en fenêtre de sécurité (r1 ; r2) et un événement ventriculaire subséquent survenant hors fenêtre de sécurité (R1, R2).

4. Le dispositif de la revendication 3, dans lequel les moyens de détection de tachycardies ventriculaires sont des moyens aptes, en cas de suspicion avérée de tachycardie ventriculaire stable, à :
- prolonger l'intervalle d'échappement auriculaire (IEA') appliqué aux moyens de délivrance d'impulsions de stimulation auriculaire, et
- confirmer la présence d'une tachycardie ventriculaire en cas d'absence d'événement ventriculaire consécutif survenant en fenêtre de sécurité.

5. Le dispositif de la revendication 4, dans lequel les moyens de détection de tachycardies ventriculaires sont des moyens aptes, après confirmation de la présence d'une tachycardie ventriculaire, à restaurer l'intervalle d'échappement auriculaire à sa valeur antérieure (IEA).

6. Le dispositif de la revendication 3, dans lequel :
- il est prévu des moyens de mesure des intervalles de couplage entre événements ventriculaires successifs (R1-r2, r2-R2, R2-r3, ...), et
- les moyens de suspicion préalable de tachycardie ventriculaire stable sont conditionnés par :
b) la vérification de la stabilité de ces intervalles de couplage mesurés.

7. Le dispositif de la revendication 3, dans lequel les moyens de suspicion préalable de tachycardie ventriculaire stable sont conditionnés par :
c) la détection de l'absence de prolongation de l'intervalle d'échappement auriculaire depuis un nombre prédéterminé de cycles antérieurs ou une durée prédéterminée antérieure.

8. Le dispositif de la revendication 3, dans lequel :
- il est prévu des moyens de mesure des intervalles de couplage entre événements ventriculaires successifs (R1-r2, r2-R2, R2-r3, ...),
- il est prévu des moyens d'analyse des arythmies ventriculaires, dans lesquels les intervalles de couplage ainsi mesurés sont comparés à un seuil de détection des tachycardies ventriculaires (TDI), et
- les moyens de suspicion préalable de tachycardie ventriculaire stable sont conditionnés par :
d) la vérification de ce que les intervalles de couplage mesurés présentent une durée inférieure audit seuil de détection des tachycardies ventriculaires.

## Claims

1. Active implantable medical device of cardiac prosthesis type for stimulation, resynchronization cardioversion and/or defibrillation, this device being designed to work in at least one AAI operating mode with ventricular detection and comprising:
- means for detecting spontaneous ventricular and auricular events;
- means for delivering auricular stimulation pulses;
- means designed, after delivery of an auricular stimulation pulse, to concurrently apply to the ventricular detection means a refractory period (PR) of predetermined duration,
this device being **characterized in that** it also comprises;
- means designed, after delivery of an auricular stimulation pulse, to concurrently apply to the ventricular detection means a safety window (FS) of predetermined duration;
- means for determining a start of a spontaneous ventricular cycle in response to a detection of a ventricular event outside the safety window (R0, R1, R2, R3);
- means for detecting ventricular tachycardia in response to the detection of ventricular events occurring both in the safety window (r1, r2, r3) and outside the safety window (R0, R1, R2, R3).

2. Device according to Claim 1, in which:
- the device is also designed to work in a DDD operating mode and comprises means designed to control the conditional switchover from the AAI mode to the DDD mode, and vice versa and
- the means for detecting ventricular tachycardia are designed to inhibit the switchover from the AAI mode to the DDD mode in case of detection of a ventricular tachycardia.

3. Device according to Claim 1, in which:
- the means for detecting ventricular tachycardia comprise means for the prior suspicion of stable ventricular tachycardia, conditioned by:
a) the detection of a sequence of events comprising, between two consecutive auricular stimulations (A1, A2; A2, A3), a ventricular event occurring in the safety window (r1, r2) and a subsequent ventricular event occurring outside the safety window (R1, R2).

4. Device according to Claim 3, in which the means for detecting ventricular tachycardia are means designed, in case of proven suspicion of stable ventricular tachycardia, to:
- prolong the auricular ejection interval (IEA') applied to the means for delivering auricular stimulation pulses, and
- confirm the presence of a ventricular tachycardia in case of absence of any consecutive ventricular event occurring in the safety window.

5. Device according to Claim 4, in which the means for detecting ventricular tachycardia are means designed, after confirmation of the presence of a ventricular tachycardia, to restore the auricular ejection interval to its previous value (IEA).

6. Device according to Claim 3, in which:
- means are provided for measuring coupling intervals between successive ventricular events (R1-r2, r2-R2, R2-r3, etc.), and
- the means for prior suspicion of stable ventricular tachycardia are conditioned by:
b) verification of the stability of these measured coupling intervals.

7. Device according to Claim 3, in which the means for prior suspicion of stable ventricular tachycardia are conditioned by:
c) the detection of the absence of extension of the auricular ejection interval from a predetermined number of previous cycles or a previous predetermined duration.

8. Device according to Claim 3, in which:
- means are provided for measuring coupling intervals between successive ventricular events (R1-r2, r2-R2, R2-r3, etc.),
- means are provided for analysing ventricular arrhythmia, in which the duly measured coupling intervals are compared to a ventricular tachycardia detection threshold (TDI), and
- the means for prior suspicion of stable ventricular tachycardia are conditioned by:
d) the verification that the measured coupling intervals have a duration less than said ventricular tachycardia detection threshold.

## Patentansprüche

1. Aktive implantierbare medizinische Vorrichtung des Typs Stimulations-, Resynchronisations-, Kardioversions- und/oder Defibrillations-Herzprothese, wobei diese Vorrichtung dazu ausgelegt ist, in wenigstens einer AAI-Betriebsart mit ventrikulärer Detektion zu arbeiten, und umfasst:
- Mittel zum Detektieren spontaner ventrikulärer und artrialer Ereignisse;
- Verabreichen artrialer Stimulationsimpulse;
- Mittel, die nach der Verabreichung eines artrialen Stimulationsimpulses konkurrent zu den ventrikulären Detektionsmitteln eine Refraktärperiode (PR) mit vorgegebener Dauer anwenden können,
wobei diese Vorrichtung **dadurch gekennzeichnet ist, dass** sie außerdem umfasst:
- Mittel, die nach der Verabreichung eines artrialen Stimulationsimpulses konkurrent zu den ventrikulären Detektionsmitteln ein Sicherheitsfenster (FS) mit vorgegebener Dauer anwenden können;
- Mittel zum Bestimmen eines Beginns eines spontanen ventrikulären Zyklus in Reaktion auf eine Detektion eines ventrikulären Ereignisses außerhalb des Sicherheitsfensters (R0, R1, R2, R3);
- Mittel zum Detektieren ventrikulärer Tachykardien in Reaktion auf die Detektion ventrikulärer Ereignisse, die sowohl im Sicherheitsfenster (r1, r2, r3) als auch außerhalb des Sicherheitsfensters (R0, R1, R2, R3) auftreten.

2. Vorrichtung nach Anspruch 1, wobei:
- die Vorrichtung auch dazu ausgelegt ist, in einer DDD-Betriebsart zu arbeiten, und Mittel enthält, die dazu ausgelegt sind, den bedingten Wechsel von der AAI-Betriebsart zu der DDD-Betriebsart und umgekehrt zu steuern, und
- die Mittel für die Detektion ventrikulärer Tachykardien dazu ausgelegt sind, den Wechsel von der AAI-Betriebsart in die DDD-Betriebsart im Fall der Detektion einer ventrikulären Tachykardie zu verhindern.

3. Vorrichtung nach Anspruch 1, wobei:
- die Mittel zum Detektieren ventrikulärer Tachykardien Mittel zum vorherigen Vermuten einer stabilen ventrikulären Tachykardie umfassen, die konditioniert sind durch:
a) die Detektion einer Folge von Ereignissen, die zwischen zwei aufeinander folgenden artrialen Stimulationen (A1, A2; A2, A3) ein ventrikuläres Ereignis, das in dem Sicherheitsfenster (r1; r2) auftritt, und ein nachfolgendes ventrikuläres Ereignis, das außerhalb des Sicherheitsfensters (R1, R2) auftritt, enthält.

4. Vorrichtung nach Anspruch 3, wobei die Mittel zum Detektieren ventrikulärer Tachykardien Mittel sind, die im Fall einer sich als richtig erweisenden Vermutung einer stabilen ventrikulären Tachykardie in der Lage sind:
- das artriale Ausreißerintervall (IEA'), das in die Mittel zum Verabreichen artrialer Stimulationsimpulse eingegeben wird, zu verlängern und
- das Vorliegen einer ventrikulären Tachykardie zu bestätigen, falls ein im Sicherheitsfenster auftretendes nachfolgendes ventrikuläres Ereignis fehlt.

5. Vorrichtung nach Anspruch 4, wobei die Mittel zum Detektieren ventrikulärer Tachykardien Mittel sind, die nach der Bestätigung des Vorhandenseins einer ventrikulären Tachykardie in der Lage sind, das artriale Ausreißerintervall auf seinen vorherigen Wert (IEA) wiederherzustellen.

6. Vorrichtung nach Anspruch 3, wobei:
- Mittel zum Messen der Kopplungsintervalle zwischen aufeinander folgenden ventrikulären Ereignissen (R1-r2, r2-R2, R2-r3, ...) vorgesehen sind und
- die Mittel zum vorherigen Vermuten einer stabilen ventrikulären Tachykardie konditioniert sind durch:
b) die Verifikation der Stabilität dieser gemessenen Kopplungsintervalle.

7. Vorrichtung nach Anspruch 3, wobei die Mittel zum vorherigen Vermuten einer stabilen ventrikulären Tachykardie konditioniert sind durch:
c) die Detektion des Fehlens der Verlängerung des artrialen Ausreißerintervalls seit einer vorgegebenen Anzahl früherer Zyklen oder einer früheren vorgegebenen Dauer.

8. Vorrichtung nach Anspruch 3, wobei:
- Mittel zum Messen der Kopplungsintervalle zwischen aufeinander folgenden ventrikulären Ereignissen (R1-r2, r2-R2, R2-r3, ...) vorgesehen sind,
- Mittel zum Analysieren ventrikulärer Arrhythmien vorgesehen sind, in denen die somit gemessenen Kopplungsintervalle mit einem Detektionsschwellenwert für ventrikuläre Tachykardien (TDI) verglichen werden, und
- die Mittel zum vorherigen Vermuten einer stabilen ventrikulären Tachykardie konditioniert sind durch:
d) die Verifikation, dass die gemessenen Kopplungsintervalle eine Dauer besitzen, die kleiner ist als der Detektionsschwellenwert für ventrikuläre Tachykardien.
